# EUROPEAN PATENT APPLICATION

(11) **EP 2 662 025 A1**
(43) Date of publication of application: **13.11.2013**
(21) Application number: 13166834.5
(22) Date of filing: 07.05.2013
(51) Int. Cl.: A61B 8/00, A61B 8/08

(54) **Ultrasonic diagnostic apparatus and control method thereof**

(30) Priority: 07.05.2012 KR 20120048127
(71) Applicant: Samsung Electronics Co., Ltd, Gyeonggi-do 443-742 (KR); Samsung Medison Co., Ltd., Hongcheon-gun, Gangwon-do (KR)
(72) Inventor: Chang, Eun Jung, Gyeonggi-do (KR); Lee, Jin Yong, Seoul (KR)
(74) Representative: Grünecker, Kinkeldey, Stockmair & Schwanhäusser

(57) **Abstract**

An ultrasonic diagnostic apparatus and a control method thereof include a probe to emit an ultrasonic signal to an object and receive the ultrasonic signal reflected from the object, a first mode image generator to generate an image of a first mode based on the ultrasonic signal received by the probe, a second mode image generator to generate an image of a second mode associated with the first mode image, a display unit to display the first mode image and the second mode image on one screen thereof, and a display controller to, when a part of a portion of the first mode image associated with the second mode image is not displayed on the display unit, move the first mode image displayed on the display unit to fully display the portion associated with the second mode image on the display unit.

## Description

### BACKGROUND OF THE INVENTION

### 1. Field of the Invention

The present invention relates to ultrasonic imaging, and in particular to an ultrasonic diagnostic apparatus which simultaneously displays two types of data on one screen, and a control method thereof.

### 2. Description of the Related Art

An ultrasonic diagnostic apparatus emits an ultrasonic signal to the surface of an object toward a target region within the body of the object and non-invasively obtains a tomographic image of soft tissue or an image of a blood stream using information of a reflected ultrasonic signal as an ultrasonic echo signal.

The ultrasonic diagnostic apparatus is small in size and low in cost, displays an image in real-time and provides high safety owing to no exposure of a subject or patient to an x-ray or other potentially dangerous forms of radiation, such as positrons, etc., as compared with other image diagnostic apparatuses such as an x-ray diagnostic apparatus, an x-ray computed tomography (CT) scanner, a magnetic resonance imager (MRI) and a nuclear medicine diagnostic apparatus. In this regard, the ultrasonic diagnostic apparatus is widely used for a heart diagnosis, an abdominal diagnosis, a urological diagnosis and an obstetrical and gynecological diagnosis.

When a diagnosis is made using the ultrasonic diagnostic apparatus, two or more types of data may be simultaneously displayed on one screen such that the diagnosis is efficiently made. For example, an image of a B-mode, which is an ultrasonic diagnostic mode, and an image of an M-mode, which is another ultrasonic diagnostic mode, may be simultaneously displayed, and a diagnosis may be made based on a comparison between those images. In this case, the B-mode image may be displayed while being reduced in size, or a part thereof may not be displayed on the screen, resulting in inaccuracy in the diagnosis.

### SUMMARY OF THE INVENTION

Therefore, it is an aspect of the present invention to provide an ultrasonic diagnostic apparatus which, when displaying two or more types of ultrasonic data on one screen, prevents the data from being reduced in size or being omitted on the screen, and a control method thereof.

It is another aspect of the present invention to provide an ultrasonic diagnostic apparatus which displays data omitted on a screen through a simple operation of the user, thereby reducing the number of operation steps of the user, and allows the user to perform data comparison/analysis by intuition, and a control method thereof.

Additional aspects of the invention will be set forth in part in the description which follows and, in part, will be obvious from the description, or may be learned by practice of the invention.

In accordance with one aspect of the present invention, an ultrasonic diagnostic apparatus includes a probe to emit an ultrasonic signal to an object and receive the ultrasonic signal reflected from the object, a first mode image generator to generate an image of a first mode based on the ultrasonic signal received by the probe, a second mode image generator to generate an image of a second mode associated with the first mode image, a display unit to display the first mode image and the second mode image on one screen thereof, and a display controller to, when a part of a portion of the first mode image associated with the second mode image is not displayed on the display unit, move the first mode image displayed on the display unit to fully display the portion associated with the second mode image on the display unit.

The first mode and the second mode refer to the mode/type of imaging performed by the ultrasonic diagnostic apparatus. In particular, the first mode may be different from the second mode. The expression "not displayed on the display unit" is to be understood in the same way as "omitted on the display unit" or "hidden on the display unit" meaning that a part of an ultrasonic image does not appear or initially appear on the display unit, although the image data may be available and stored in memory, such as a display buffer for ready access and display on the display unit.

The first mode image may be a B-mode image having at least one M line marked therein, and the second mode image may be an M-mode image corresponding to the at least one M line marked in the B-mode image.

The display controller, when a portion of the M line is not displayed on the display unit, may move the B-mode image to display the M line portion on the display unit.

The ultrasonic diagnostic apparatus may further include an input unit to receive a user command for movement of the B-mode image displayed on the display unit, wherein the display controller may move the B-mode image displayed on the display unit based on a moving direction and a moving amount corresponding to the user command received from the input unit.

The display unit may display a pointing tool, with the pointing tool being moved in response to the user command received from the input unit, wherein the display controller, when a portion of the M line located in a moving direction of the pointing tool is not displayed on the display unit, may move the B-mode image displayed on the display unit to display the M line portion on the display unit.

Alternatively, the display unit may display a pointing tool, with the pointing tool being moved in response to the user command received from the input unit, wherein the display controller, when a portion of the M line corresponding to a portion of the M-mode image in which the pointing tool moved in response to the user command is located is not displayed on the display unit, may move the B-mode image to display the M line portion on the display unit.

The ultrasonic diagnostic apparatus may further include an input unit to receive a setting of the M line input by a user, wherein the display controller, when an additional setting of an M line is received from the input unit and a portion of the additionally set M line is not displayed on the display unit, may move the B-mode image displayed on the display unit to fully display the additionally set M line.

The display controller may additionally display an M-mode image corresponding to the additionally set M line on the display unit when the additional M line setting is completed.

The display controller may rotate the B-mode image such that the M line marked in the B-mode image is parallel with a vertical axis of the M-mode image.

The display controller may scale the B-mode image displayed on the display unit such that a depth ratio of the B-mode image to the M-mode image is 1:1.

In accordance with another aspect of the present invention, an ultrasonic diagnostic apparatus includes a probe to emit an ultrasonic signal to an object and receive the ultrasonic signal reflected from the object, a first mode image generator to generate at least one two-dimensional ultrasonic image of the object based on the ultrasonic signal received by the probe, a second mode image generator to generate a three-dimensional ultrasonic image of the object, a display unit to display the two-dimensional ultrasonic image and the three-dimensional ultrasonic image on one screen thereof, a display controller to, when any one of the two-dimensional ultrasonic image and the three-dimensional ultrasonic image is moved, move the other image corresponding to the image movement, and an input unit to receive a user command for the image movement.

The display controller may move the two-dimensional ultrasonic image displayed on the display unit based on a moving direction and a moving amount corresponding to the user command received from the input unit and move the three-dimensional ultrasonic image displayed on the display unit together corresponding to the movement of the two-dimensional ultrasonic image.

Alternatively, the display controller may move the three-dimensional ultrasonic image displayed on the display unit based on a moving direction and a moving amount corresponding to the user command received from the input unit and move the two-dimensional ultrasonic image displayed on the display unit together corresponding to the movement of the three-dimensional ultrasonic image.

The input unit may include at least one of a mouse, a trackball, a keyboard, a touch panel and a touch screen.

In accordance with another aspect of the present invention, an ultrasonic diagnostic apparatus includes a probe to emit an ultrasonic signal to an object and receive the ultrasonic signal reflected from the object, a B-mode image generator to generate a B-mode image based on the ultrasonic signal received by the probe, an input unit to receive a setting of an M line in the B-mode image, an M-mode image generator to generate an M-mode image corresponding to the set M line, a display unit to display the B-mode image and the M-mode image on one screen thereof, and a display controller to, when a portion of the set M line is not displayed on the display unit during the M line setting, move the B-mode image to fully display the set M line on the display unit.

In accordance with another aspect of the present invention, a method of controlling an ultrasonic diagnostic apparatus includes emitting an ultrasonic signal to an object and receiving the ultrasonic signal reflected from the object, generating an image of a first mode based on the received ultrasonic signal and displaying the generated first mode image, generating an image of a second mode associated with the first mode image and displaying the generated second mode image together with the first mode image, and moving the displayed first mode image when a part of a portion of the first mode image associated with the second mode image is not displayed, to fully display the portion associated with the second mode image.

The first mode image may be a B-mode image having at least one M line marked therein, and the second mode image may be an M-mode image corresponding to the at least one M line.

The moving of the displayed first mode image may include moving the B-mode image when a portion of the M line is not displayed, to display the M line portion.

The method may further include receiving a user command for image movement, wherein the moving of the displayed first mode image may include moving the displayed B-mode image based on a moving amount and a moving direction corresponding to the received user command.

The method may further include displaying a pointing tool, with the pointing tool being moved in response to the received user command, wherein the moving of the displayed first mode image may include moving the B-mode image when a portion of the M line located in a moving direction of the pointing tool is not displayed, to display the M line portion.

Alternatively, the method may further include displaying a pointing tool, with the pointing tool being moved in response to the received user command, wherein the moving of the displayed first mode image may include moving the B-mode image when a portion of the M line corresponding to a portion of the M-mode image in which the pointing tool moved in response to the user command is located is not displayed, to display the M line portion.

The method may further include receiving an additional setting of an M line input by a user, wherein the moving of the displayed first mode image may include moving the B-mode image when a portion of the additionally set M line is not displayed, to fully display the additionally set M line.

The moving of the displayed first mode image may include rotating the B-mode image such that the M line marked in the B-mode image is parallel with a vertical axis of the M-mode image.

The method may further include scaling the displayed B-mode image such that a depth ratio of the B-mode image to the M-mode image is 1:1.

In accordance with another aspect of the present invention, a method of controlling an ultrasonic diagnostic apparatus includes emitting an ultrasonic signal to an object and receiving the ultrasonic signal reflected from the object, generating at least one two-dimensional ultrasonic image of the object based on the received ultrasonic signal, generating a three-dimensional ultrasonic image of the object, displaying the two-dimensional ultrasonic image and the three-dimensional ultrasonic image on one screen, and moving, when any one of the two-dimensional ultrasonic image and the three-dimensional ultrasonic image is moved, the other image corresponding to the image movement.

The method may further include receiving a user command for the image movement, wherein the moving of the other image may include moving, when the displayed two-dimensional ultrasonic image is moved based on a moving direction and a moving amount corresponding to the received user command, the displayed three-dimensional ultrasonic image together corresponding to the movement of the two-dimensional ultrasonic image.

Alternatively, the method may further include receiving a user command for the image movement, wherein the moving of the other image may include moving, when the displayed three-dimensional ultrasonic image is moved based on a moving direction and a moving amount corresponding to the received user command, the displayed two-dimensional ultrasonic image together corresponding to the movement of the three-dimensional ultrasonic image.

In accordance with a further aspect of the present invention, a method of controlling an ultrasonic diagnostic apparatus includes emitting an ultrasonic signal to an object and receiving the ultrasonic signal reflected from the object, generating a B-mode image based on the received ultrasonic signal and displaying the generated B-mode image, receiving a setting of an M line in the B-mode image, and moving the B-mode image when a portion of the set M line is not displayed during the M line setting, to fully display the set M line.

The method may further include generating an M-mode image corresponding to the set M line when the M line setting is completed, and then displaying the generated M-mode image together with the B-mode image.

### BRIEF DESCRIPTION OF THE DRAWINGS

These and/or other aspects of the invention will become apparent and more readily appreciated from the following description of the embodiments, taken in conjunction with the accompanying drawings of which:
FIG. 1 is a block diagram showing the configuration of an ultrasonic diagnostic apparatus according to an exemplary embodiment of the present invention;
FIG. 2 is a view illustrating a plurality of scan lines along which ultrasonic signals from a probe in FIG. 1 are transmitted;
FIGS. 3A and 3B are views illustrating methods of simultaneously displaying a B-mode image and an M-mode image on a screen by an ultrasonic diagnostic apparatus in the prior art;
FIG. 4 is a block diagram of the ultrasonic diagnostic apparatus in greater detail according to the exemplary embodiment of the present invention;
FIG. 5 is a view illustrating images of a first example displayed on a display unit of the ultrasonic diagnostic apparatus according to the exemplary embodiment of the present invention;
FIGS. 6A and 6B are views illustrating images of a second example displayed on the display unit of the ultrasonic diagnostic apparatus according to the exemplary embodiment of the present invention;
FIGS. 7A and 7B are views illustrating images of a third example displayed on the display unit of the ultrasonic diagnostic apparatus according to the exemplary embodiment of the present invention;
FIG. 8 is a view illustrating images of a fourth example displayed on the display unit of the ultrasonic diagnostic apparatus according to the exemplary embodiment of the present invention;
FIG. 9 is a view illustrating images of a fifth example displayed on the display unit of the ultrasonic diagnostic apparatus according to the exemplary embodiment of the present invention;
FIG. 10 is a view illustrating images of a sixth example displayed on the display unit of the ultrasonic diagnostic apparatus according to the exemplary embodiment of the present invention;
FIG. 11 is a view illustrating examples of images displayed on a display unit of an ultrasonic diagnostic apparatus according to an alternative exemplary embodiment of the present invention;
FIG. 12 is a flowchart illustrating a method of controlling the ultrasonic diagnostic apparatus according to the exemplary embodiment of the present invention;
FIG. 13 is a flowchart illustrating an example of automatic image movement;
FIG. 14 is a flowchart illustrating an example of movement of a B-mode image based on the position of a pointing tool;
FIG. 15 is a flowchart illustrating an example of movement of the B-mode image based on an additional setting of an M line; and
FIG. 16 is a flowchart illustrating an example of rotation and scaling of the B-mode image.

### DETAILED DESCRIPTION OF THE INVENTION

Reference will now be made in detail to exemplary embodiments of the present invention, examples of which are illustrated in the accompanying drawings, wherein like reference numerals refer to like elements throughout. Exemplary embodiments of the present invention will be described with reference to the accompanying drawings. In the following description, a detailed explanation of known related functions and constructions may be omitted to avoid unnecessarily obscuring the subject matter of the present invention. The present invention may, however, be embodied in many different forms and should not be construed as limited to the exemplary embodiments set forth herein. In addition, terms described herein, which are defined with reference to the functions of the present invention, may be implemented differently depending on a user or operator's intention and practice. Therefore, the terms should be understood on the basis of the disclosure throughout the specification. The principles and features of this invention may be employed in varied and numerous embodiments without departing from the scope of the invention.

Furthermore, although the drawings represent exemplary embodiments of the invention, the drawings are not necessarily to scale and certain features may be exaggerated or omitted in order to more clearly illustrate and explain the present invention.

Among the terms set forth herein, a screen refers to a display or other output devices which visually display information to the user, and which optionally are capable of receiving and electronically processing tactile inputs from a user using a stylo, a finger of the user, or other techniques for conveying a user selection from the user to the output devices.

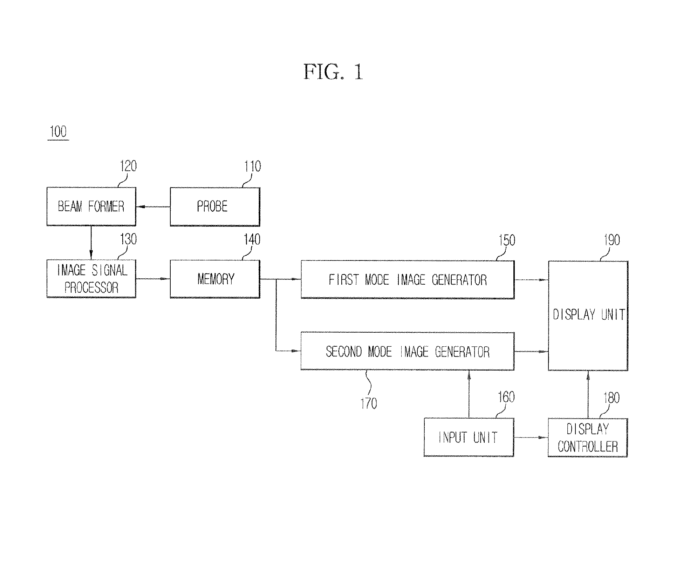

FIG. 1 is a block diagram showing the configuration of an ultrasonic diagnostic apparatus according to an exemplary embodiment of the present invention.

Referring to FIG. 1, the ultrasonic diagnostic apparatus 100 according to the exemplary embodiment of the present invention includes a probe 110, a beam former 120, an image signal processor 130, a memory 140, a first mode image generator 150, a second mode image generator 170, a display unit 190, an input unit 160, and a display controller 180.

The probe 110 includes a plurality of transducer elements to convert ultrasonic signals and electrical signals into each other. Each of the transducer elements transmits an ultrasonic signal to an object and receives the ultrasonic signal as an ultrasonic echo signal reflected from the object to generate a receive signal. Here, the receive signal is an analog signal.

In particular, the probe 110 transmits an ultrasonic beam focused by appropriately delaying input times of pulses to the respective transducer elements to the object along transmission scan lines. On the other hand, ultrasonic echo signals reflected from the object are input at different input times to the respective transducer elements, which then output the received ultrasonic echo signals.

When the probe 110 transmits ultrasonic signals, the beam former 120 adjusts driving timings of the respective transducer elements of the probe 110 to focus the ultrasonic signals at a specific point. Also, upon receiving the receive signals of an analog form from the probe 110, the beam former 120 converts the receive signals into digital signals. Then, the beam former 120 focuses the digital signals in consideration of the positions of the transducer elements and the focusing point to generate a receive focus signal.

The image signal processor 130 performs an envelope detection process of detecting the magnitude of an ultrasonic signal, based on an ultrasonic signal focused by the beam former 120, to generate ultrasonic image data.

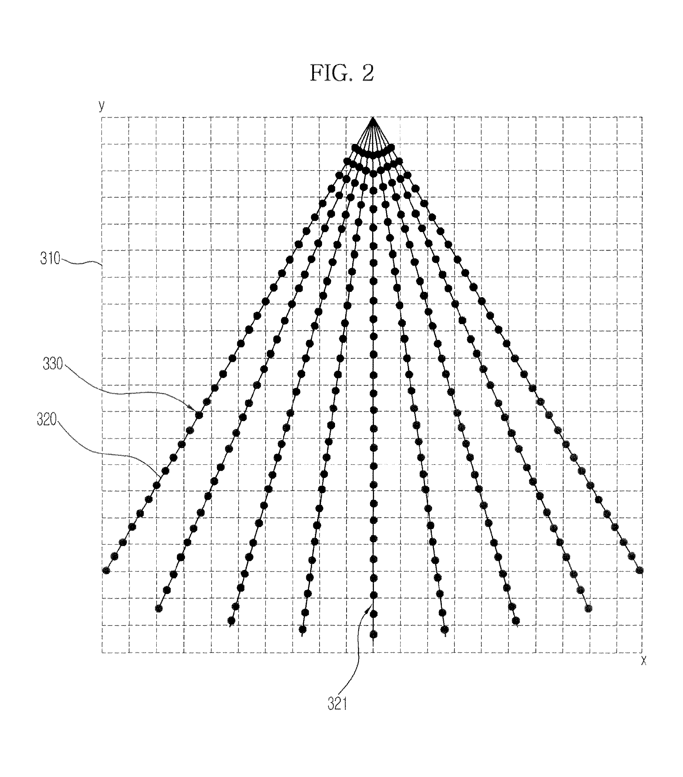

FIG. 2 illustrates a plurality of scan lines 320 along which ultrasonic signals from the probe 110 are transmitted. Referring to FIG. 2, a plurality of points 330 are present on each of the scan lines 320. The image signal processor 130 generates ultrasonic image data based on information about the positions of the points 330 on each scan line 320 and data obtained from each of the points 330. The ultrasonic image data includes information about coordinates of each point 330 on, for example, an x-y coordinate system 310, information about the angle of each scan line 320 relative to a vertical scan line 321, the data obtained from each point 330, etc. The generated ultrasonic image data is stored in the memory 140.

Referring again to FIG. 1, in addition to generating the ultrasonic image data, the image signal processor 130 performs a logarithmic amplitude process to increase a relative brightness difference corresponding to a dark portion of an ultrasonic image, and reduce a relative brightness difference corresponding to a bright portion of the ultrasonic image.

In particular, when a brightness mode (B-mode) image is generated based on an ultrasonic signal focused by the beam former 120, the generated B-mode image may be poor in quality due to an excessive brightness difference between a bright portion and a dark portion thereof. In this case, only the bright portion may be distinctly displayed or only the dark portion may be distinctly displayed.

In this regard, the image signal processor 130 performs the logarithmic amplitude process to increase the relative brightness difference of the dark portion and reduce the relative brightness difference of the bright portion, so as to generate a fine B-mode image.

The ultrasonic diagnostic apparatus 100 according to the exemplary embodiment of the present invention may display two or more types of data on one screen. Here, the two or more types of data may be image data acquired respectively in different diagnostic modes of the ultrasonic diagnostic apparatus 100, which may be of various known types including a motion mode (M-mode), a B-mode, a Doppler mode, and a color flow mapping mode. Also, the diagnostic modes of the ultrasonic diagnostic apparatus 100 may include a two-dimensional mode and a three-dimensional mode.

The B-mode is used to display a cross-sectional image of the inside of an object. In the B-mode, a portion of the image having a strong reflected echo and a portion of the image having a weak reflected echo are represented by a brightness difference. The image of the B-mode is configured based on information obtained from several tens to hundreds of scan lines.

The M-mode is used to display an image indicative of how biometrics information (for example, luminance information) about a specific portion (a motion line; that is, an M line) of the cross-sectional image (B-mode image) of the object varies with time. In general, the image of the B-mode and the image of the M-mode are simultaneously displayed on one screen so that an examiner may make an accurate diagnosis through comparison/analysis of the two types of data.

The Doppler mode uses a Doppler effect which is a change in frequency of sound emitted from a moving object. In particular, a color Doppler mode provides a power image indicative of a two-dimensional distribution of the power of a Doppler signal, and a velocity image indicative of a two-dimensional distribution of the velocity of the Doppler signal. The images of the color Doppler mode may not only visualize a blood stream in real-time, but also express the states of a wide range of blood streams, from a blood stream of a high velocity in a large blood vessel to a blood stream of a low velocity in a small blood vessel. The M-mode image may also be generated with respect to the Doppler mode images.

The first mode image generator 150 generates an ultrasonic image in a first mode which is one of the above diagnostic modes, and the second mode image generator 170 generates an ultrasonic image in a second mode which is another one of the above diagnostic modes.

In the case where the first mode is the B-mode and the second mode is the M-mode, the display unit 190 simultaneously displays a B-mode image and an M-mode image corresponding to an M line set in the B-mode image. The first mode and the second mode may be interchangeable.

In the case where the first mode is the color Doppler mode and the second mode is the M-mode, the display unit 190 simultaneously displays a color Doppler mode image and a color M-mode image set in the color Doppler mode image. The first mode and the second mode may be interchangeable.

In the case where the first mode is the two-dimensional mode and the second mode is the three-dimensional mode, the display unit 190 simultaneously displays a two-dimensional ultrasonic image and a three-dimensional ultrasonic image. The first mode and the second mode may be interchangeable.

The ultrasonic diagnostic apparatus 100 according to the exemplary embodiment of the present invention may be particularly usefully applied in simultaneously displaying a B-mode image and an M-mode image. Hereinafter, the operation of an ultrasonic diagnostic apparatus 100 according to the exemplary embodiment of the present invention will be described in detail using the example, for illustrative purposes only, in which the first mode is the B-mode and the second mode is the M-mode.

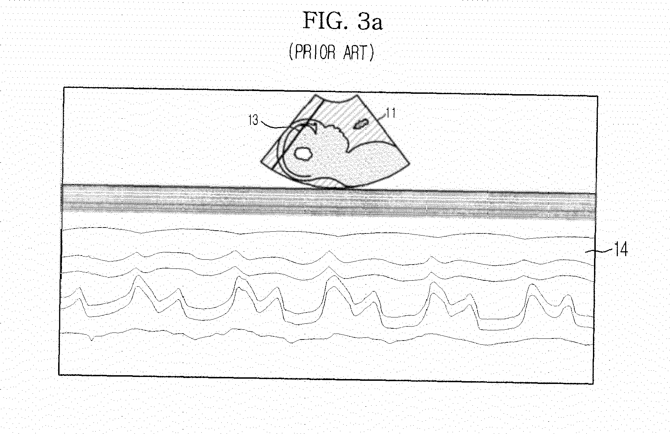

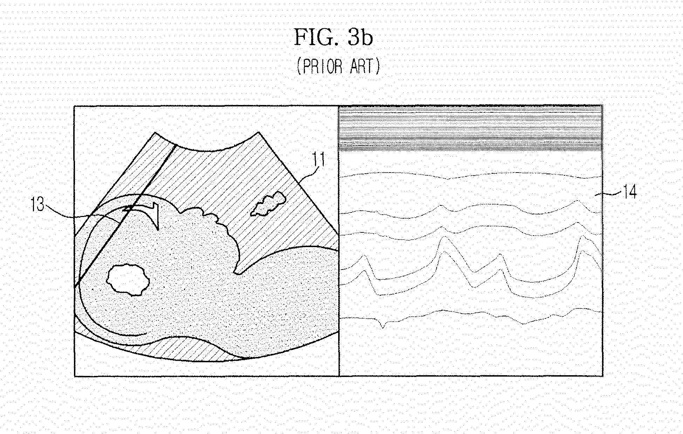

FIGS. 3A and 3B illustrate methods of simultaneously displaying a B-mode image and an M-mode image on a screen of a display unit by an ultrasonic diagnostic apparatus in the prior art.

FIG. 3A illustrates one method of simultaneously displaying a B-mode image and an M-mode image. Referring to FIG. 3A, the ultrasonic diagnostic apparatus in the prior art displays a B-mode image 11 and an M-mode image 14 on one screen with the images arranged vertically. Here, the M-mode image 14 represents biometrics information about a specific M line 13 of the B-mode image 11.

In this case, the B-mode image 11 is excessively scaled down, that is, reduced as shown in FIG. 3A in order to fit on one screen with the larger M-mode image 14, and therefore it may not be easy for the examiner to campare/analyze the B-mode image 11 and the M-mode image 14.

FIG. 3B illustrates another method of simultaneously displaying a B-mode image and an M-mode image. Referring to FIG. 3B, the ultrasonic diagnostic apparatus in the prior art displays a B-mode image 11 and an M-mode image 14 on one screen with the images 11, 14 arranged horizontally.

In this case, as shown in FIG. 3B, the B-mode image 11 is not scaled down, but at least one part thereof is omitted on the screen, such as the lower left and lower right corners of the image 11, which are visible in FIG. 3A. Therefore, the omitted at least one part of the image 11 may fail to be used for a diagnosis, which may impair or reduce the quality of the diagnosis. As a result, it may be difficult for the examiner to accurately compare/analyze the B-mode image 11 and the M-mode image 14.

Therefore, the ultrasonic diagnostic apparatus 100 according to the exemplary embodiment of the present invention moves a B-mode image displayed on a screen to show a part of the B-mode image omitted on the screen, so as to simultaneously display the B-mode image and an M-mode image without scaling-down and without partial omission of the B-mode image.

As used herein, in the exemplary embodiment to be described below, an expression "omitted on the screen", an expression "not displayed or shown on the screen", and an expression "hidden on the screen" will all be used to indicate that a part of an ultrasonic image does not appear or initially appear on the screen, although the image data may be available and stored in memory, such as a display buffer for ready access and display on the screen.

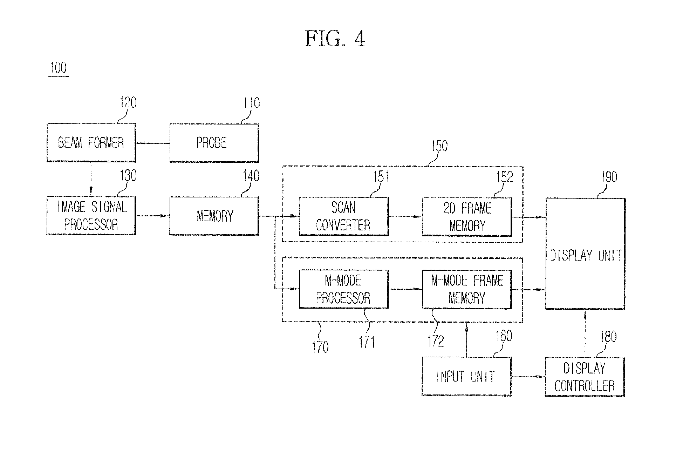

FIG. 4 is a block diagram of the ultrasonic diagnostic apparatus 100 in greater detail according to the exemplary embodiment of the present invention.

Referring to FIG. 4, the ultrasonic diagnostic apparatus 100 according to the exemplary embodiment of the present invention includes the probe 110, the beam former 120, the image signal processor 130, the memory 140, the B-mode image generator 150, the M-mode image generator 170, the display unit 190, the input unit 160, and the display controller 180.

The probe 110, the beam former 120, the image signal processor 130, and the memory 140 are the same as those components described previously with reference to FIG. 1, and a description thereof will thus be omitted.

The B-mode image generator 150 generates a cross-sectional image of an object using ultrasonic image data stored in the memory 140. To this end, the B-mode image generator 150 includes a scan converter 151 and a two-dimensional (2D) frame memory 152.

The scan converter 151 reads the ultrasonic image data stored in the memory 140, converts the format of the read data into a B-mode image format and stores the resulting data in the 2D frame memory 152.

The display unit 190 displays the B-mode image stored in the 2D frame memory 152. At this time, the display unit 190 may display an M line, which forms the basis of an M-mode image, together on the B-mode image. The display unit 190 includes a screen, which may be implemented with a liquid crystal display (LCD), a light emitting diode (LED), a touch screen, or the like.

The input unit 160 may receive a command for movement of the B-mode image or a setting of the M line, input from the user. The input unit 160 may be implemented with a keyboard, a trackball, a mouse, a touch panel, or the like provided in the ultrasonic diagnostic apparatus 100. In an exemplary embodiment in which the display unit 190 is implemented with a touch screen, the touch screen may perform both the functions of the display unit 190 and input unit 160.

The setting of the M line may be made by an input from the user or directly by the M-mode image generator 170. If the M line is set, the M-mode image generator 170 generates an M-mode image of the set M line based on the ultrasonic image data stored in the memory 140.

The M-mode image generator 170 includes an M-mode processor 171 and an M-mode frame memory 172. The M-mode processor 171 analyzes the position, angle, etc. of the set M line, generates M-mode image data based on results of the analysis and stores the generated M-mode image data in the M-mode frame memory 172. The M-mode frame memory 172 and the 2D frame memory 152 may be implemented by a single memory.

An example of generating the M-mode image data will hereinafter be described in detail. First, the range of the set M line is analyzed and a predetermined number of points on the M line are sampled. Virtual lines which proceed toward the probe 110 via the respective sampled points are set, and the angle between each of the virtual lines and a vertical scan line and the distance from the surface of the probe 110 to each of the sampled points are calculated, for example, using the configuration of the scan lines 320, the points 330, and the vertical scan line 321 on the x-y coordinate system 310 shown in FIG. 2.

Then, a plurality of points on the scan lines adjacent to each of the sampled points are retrieved from the memory 140 and respective adjacencies between each sampled point and the adjacent points are calculated. Ultrasonic image data corresponding to the adjacent points is read from the memory 140 and M-mode image data of each sampled point is generated by an interpolation based on the calculated adjacencies and the read ultrasonic image data.

The M-mode image data stored in the M-mode frame memory 172 is displayed as an M-mode image on the display unit 190. In one example implementation, the M-mode image may be displayed at one side of the B-mode image displayed on the display unit 190, so that both of the two images appear on one screen, as shown in FIG. 3B.

As stated previously with reference to FIG. 3B, when the B-mode image 11 and the M-mode image 14 are simultaneously displayed on one screen, a part of the B-mode image 11, more particularly lower left and right portions of the B-mode image 11, may be omitted on the screen. In this case, the display controller 180 of the present invention may move the displayed B-mode image 11 to display the omitted part thereof on the screen. The movement of the B-mode image 11 by the present invention is performed by up, down, left and/or right panning of the image.

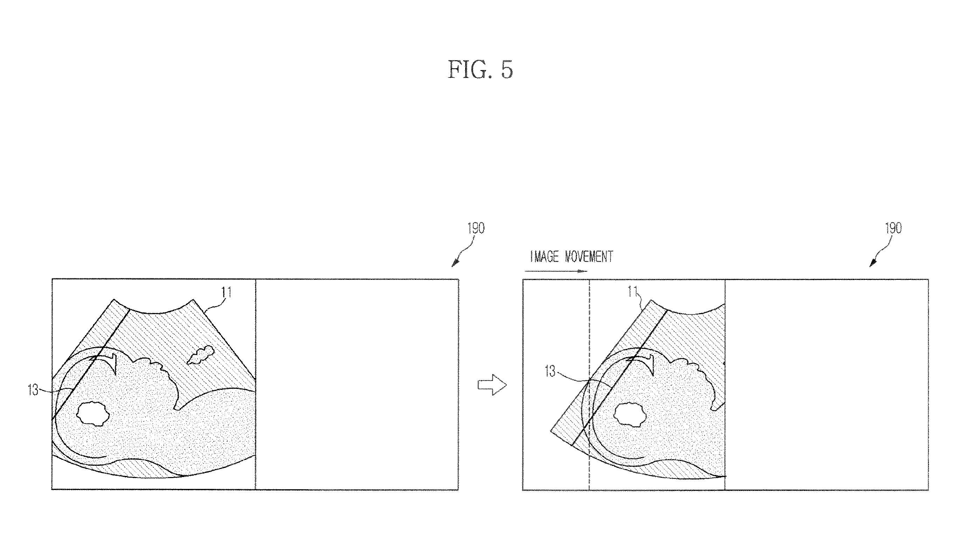

Hereinafter, examples of the operation of the display controller 180, such as the movement of the B-mode image will be described in detail with reference to FIGS. 5 to 11.

FIG. 5 illustrates images of a first example displayed on the display unit 190 of the ultrasonic diagnostic apparatus 100 according to the exemplary embodiment of the present invention.

When the user intends to personally draw an M line 13 on a B-mode image to set the M line 13, an area of the B-mode image in which the M line 13 is to be drawn may be partially shown as illustrated on the left side of FIG. 5.

For example, the input unit 160 may be implemented with a trackball, and the user clicks the trackball to designate a desired point (a start point of the M line 13) of the B-mode image, and then rolls the trackball to draw the M line 13 as desired. When an area of the B-mode image in which the M line 13 is to be drawn is partially shown, the display controller 180 moves the B-mode image to the right with movement of the trackball such that the area of the B-mode image in which the M line 13 is to be drawn is fully displayed on the screen, as shown on the right side ofi FIG. 5. Therefore, the user may completely observe the drawn M line 13.

Although drawing the M line 13 on the B-mode image has been applied as the M line setting method in the example of FIG. 5, the exemplary embodiment of the present invention is not limited thereto. For example, designating the position of the M line 13 in the B-mode image by other known drawing or input methods may be applied as the M line setting method. Even in this case, when the set M line 13 is not fully displayed on the screen, the B-mode image may be moved such that the set M line 13 is fully displayed.

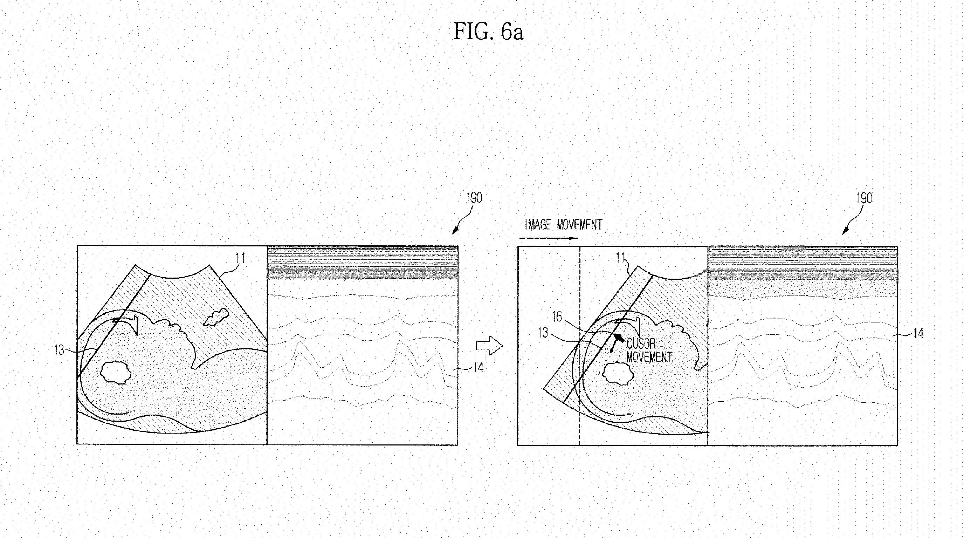

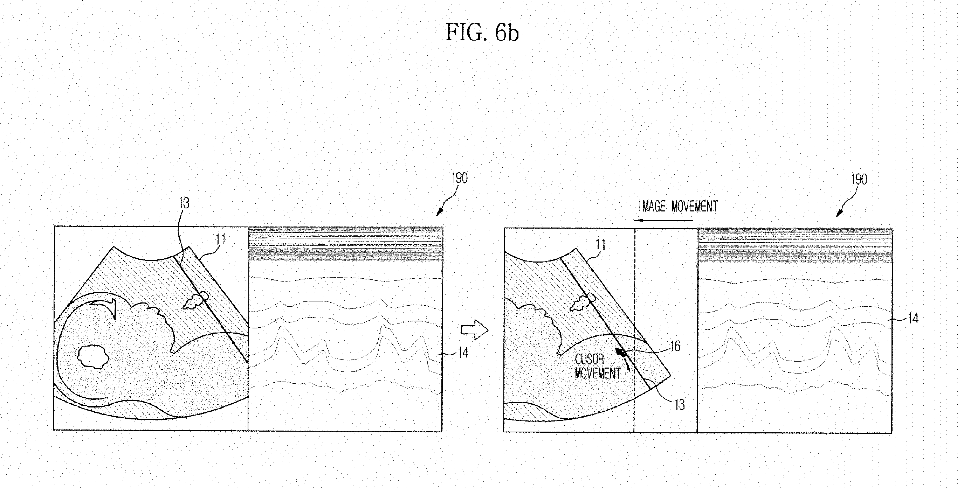

FIGS. 6A and 6B illustrate images of a second example displayed on the display unit 190 of the ultrasonic diagnostic apparatus 100 according to the exemplary embodiment of the present invention.

When the user inputs a setting of an M line 13 using the input unit 160, the display unit 190 displays a B-mode image 11 in which the M line 13 is set and marked, and displays an M-mode image 14 corresponding to the M line 13 on one screen with the B-mode image 11, as shown in FIG. 6A. At this time, a part of the B-mode image 11, more particularly lower left and right portions of the B-mode image 11, may be omitted from display on the screen, thereby causing the M line 13 to be partially shown, as illustrated on the left side of FIG. 6A.

Then, the input unit 160 receives a command for image movement input from the user. The command input is performed by manipulation of the input unit 160 by the user. In the case where the input unit 160 is implemented with a mouse or trackball, the user may input the command by moving the mouse or trackball. In the case where the input unit 160 is implemented with a touch pad or touch screen, the user may input the command by touching the touch pad or touch screen.

For example, the input unit 160 may be a trackball, a pointing tool, or a cursor 16, shown on the right side of FIG. 6A, which moves with rotation of the trackball or movement of a mouse, and is displayed on the screen of the display unit 190. If the user rotates the trackball or moves the mouse to move the cursor in the direction of the arrow in FIG. 6A, the display controller 180 moves the B-mode image 11 displayed on the screen to the right with the movement of the cursor 16 to show the left portion of the B-mode image 11 omitted on the screen, as shown at the right side of FIG. 6A. As a result, the set M line 13 is fully shown on the screen, so that the user may make an accurate diagnosis through comparison/analysis of the M line 13 marked in the B-mode image 11 and the M-mode image 14.

On the other hand, in the case where the input unit 160 is a mouse or trackball, just moving the cursor 16 on the B-mode image 11 may enable the display controller 180 to move the B-mode image 11. Alternatively, when the cursor 16 is clicked on a desired point of the B-mode image 11 and then moved, the display controller 180 may move the B-mode image 11.

There is no limitation in the direction in which the display controller 180 moves the B-mode image 11. When the M line 13 is marked in the right part of the B-mode image 11 as shown at the left side of FIG. 6B, the user may manipulate the input unit 160 to move the cursor 16 in the direction of the arrow shown at the right side of FIG. 6B. In this case, the display controller 180 may move the B-mode image 11 displayed on the screen to the left to show the right portion of the B-mode image 11 omitted from display on the screen. As a result, the set M line 13 may be fully shown on the screen.

The display controller 180 may move the B-mode image 11 based on a moving amount input using the input unit 160. Alternatively, once the user moves the cursor 16 toward an omitted portion of the M line 13 using the input unit 160, the display controller 180 may move the B-mode image 11 irrespective of the moving amount of the cursor 16 to fully show the partially omitted M line 13 on the screen. Also, the display controller 180 may move the B-mode image 11 based on a moving direction input using the input unit 160. Alternatively, the display controller 180 may move the B-mode image 11 irrespective of the input moving direction to fully show the partially omitted M line 13 on the screen.

In particular, when the user rolls the trackball 160, for example, to the left and down to move the cursor 16 to the left and down, the display controller 180 may move the B-mode image 11 in the opposite direction, that is, to the right and up.

As another alternative exemplary embodiment, the display controller 180 may move the B-mode image 11 to the right in consideration of only the moving amount irrespective of the input moving direction or irrespective of both the moving amount and the input moving direction, and accordingly to only respond to receiving any input, to fully show the partially omitted M line 13 on the screen. Notably, the reason to move the B-mode image to the left and right irrespective of the input moving direction is that only the left and right portions of the B-mode image have been omitted on the display unit 190 and both the upper and lower portions of the B-mode image have been displayed on the display unit 190. If the upper and lower portions of the B-mode image are also omitted on the display unit 190, the display controller 180 may move the B-mode image up and down, as well as to the left and right.

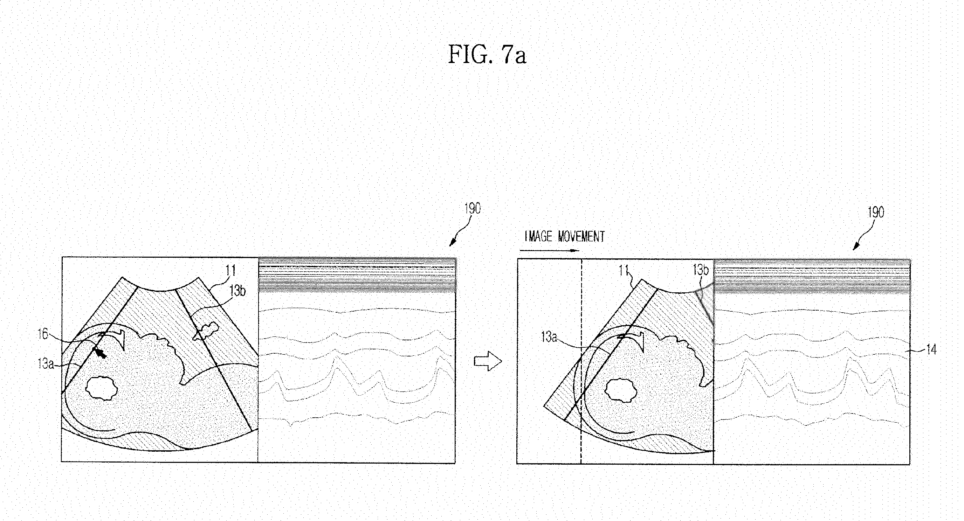

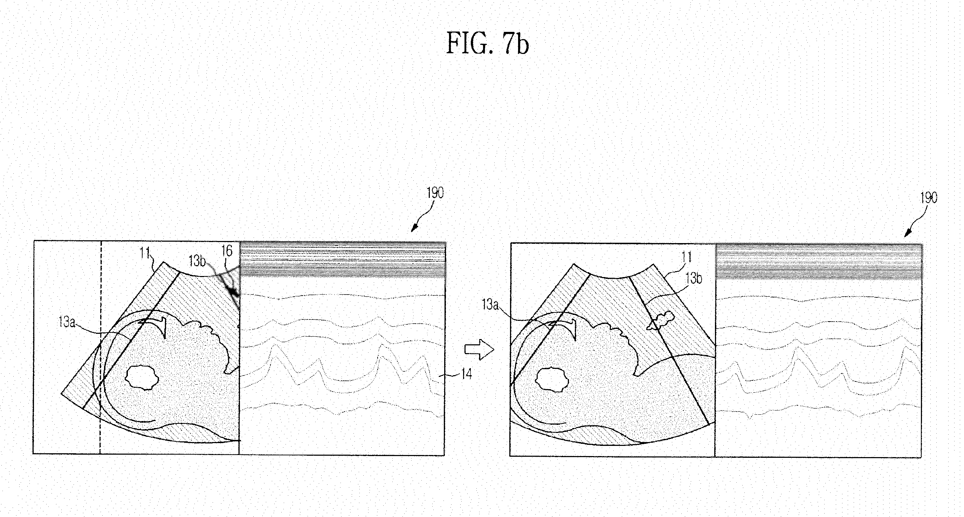

FIGS. 7A and 7B illustrate images of a third example displayed on the display unit 190 of the ultrasonic diagnostic apparatus 100 according to the exemplary embodiment of the present invention.

As shown at the left side of FIG. 7A, two M lines 13a and 13b may be set and marked with respect to a B-mode image 11, and an M-mode image 14 of the set M lines 13a and 13b may be displayed next to the B-mode image 11. The user may select the M line 13a, partially omitted on the screen, using the input unit 160 to minutely observe the partially omitted M line 13a. When the partially omitted M line 13a is selected, the display controller 180 may move the B-mode image 11, for example, to the right since the left M line 13a is inputted, to fully display the partially omitted M line 13a on the screen.

In particular, in the case where an omitted portion of the M line 13a is located in a lower left portion of the B-mode image 11 as shown at the left side of FIG. 7A, the display controller 180 may move the B-mode image 11 to the right to display the omitted lower left portion of the B-mode image 11 on the screen of the display unit 190, so as to fully display the partially omitted M line 13a on the screen, as shown at the right side of FIG. 7A.

Once the B-mode image 11 is moved, the partially omitted M line 13a is fully shown, but the other M line 13b may be partially or completely omitted as shown on the right side of FIG. 7A, or similarly on the left side of FIG. 7B. When the user selects the M line 13b using the input unit 160 to observe the M line 13b again, using the cursor 16 in FIG. 7B, the display controller 180 may move the B-mode image 11 to the left to fully display the partially omitted M line 13b on the screen, as shown at the right side of FIG. 7B.

Likewise, in the example of FIGS. 7A and 7B, the B-mode image is moved only to the left and right because the upper and lower portions thereof are not omitted on the screen. As needed, the B-mode image may be moved up, down, left and right.

Although the two M lines have been set in the example of FIGS. 7A and 7B, the exemplary embodiment of the present invention may also be applied to the case where a larger number of M lines are set and the case where only one M line is set.

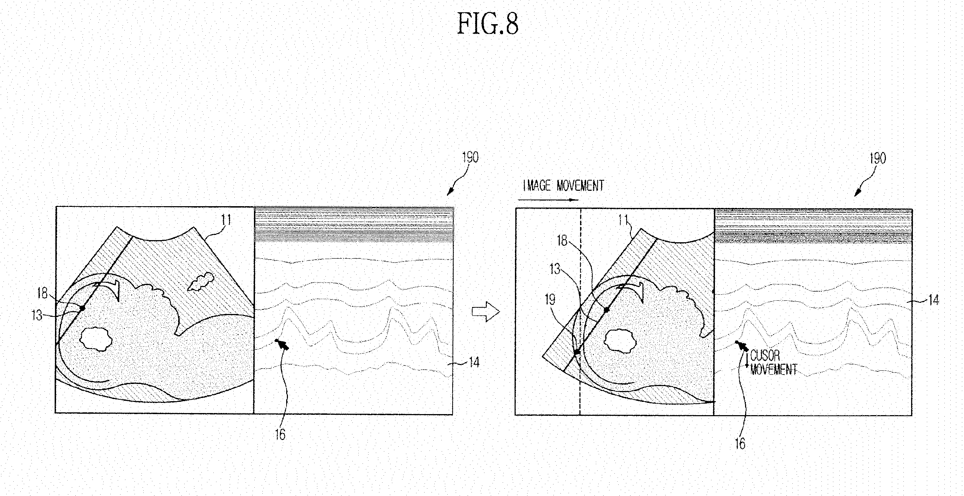

FIG. 8 illustrates images of a fourth example displayed on the display unit 190 of the ultrasonic diagnostic apparatus 100 according to the exemplary embodiment of the present invention.

Referring to the images 11, 14 shown at the left side of FIG. 8, a position 18 on the B-mode image 11 is indicated corresponding to a position of a cursor 16 on the M-mode image 14. When the user moves the position of the cursor 16 on a vertical axis of the M-mode image 14 using the input unit 160, a position 19 on the B-mode image 11 corresponding to the moved position of the cursor 16 may not be shown on the screen. In this case, the display controller 180 may move the B-mode image 11 to show the position 19 of the B-mode image 11 corresponding to the moved position of the cursor 16 on the screen. In the example of FIG. 8, the B-mode image 11 is moved to the right such that a lower left portion of the B-mode image 11, not initially shown on the screen, is shown on the screen on the right of FIG. 8.

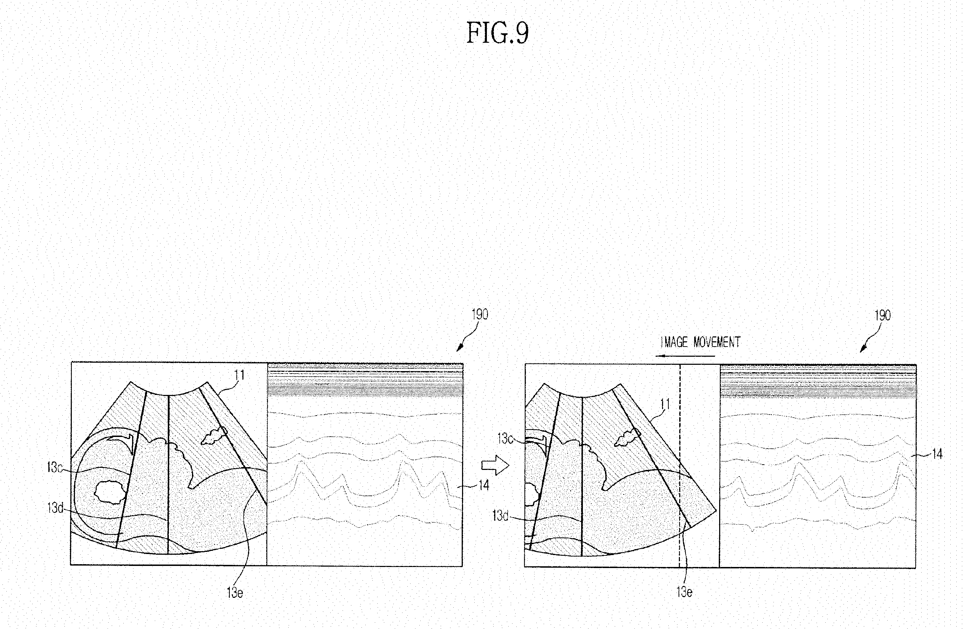

FIG. 9 illustrates images of a fifth example displayed on the display unit 190 of the ultrasonic diagnostic apparatus 100 according to the exemplary embodiment of the present invention.

Referring to FIG. 9, an M-mode image 14 corresponding to at least two M lines 13c and 13d may be displayed on the display unit 190, for example, on the left side of FIG. 9. At this time, the user may input an additional setting of an M line 13e using the input unit 160. When the M line 13e is additionally set, the M line 13e may partially appear on the screen. In this case, the display controller 180 may move the B-mode image 11 to fully show the additionally set M line 13e on the screen.

The additional setting of the M line 13e may be made by selection of the M line 13e pre-marked in the B-mode image 11 by the user, designation of the position of the M line 13e to be set by the user, or drawing of the M line 13e on the B-mode image 11 by the user.

In the example of FIG. 9, in the case where the user intends to draw the M line 13e, the user may designate, using the input unit 160, a point of the B-mode image 11 to be set as a start point of the M line 13e and then draw the M line 13e, in a similar manner to drawing the M line 13 in the example of FIG. 5. Then, the display controller 180 may move the B-mode image 11 to the left to fully display the drawn M line 13e on the screen, as shown in the right side of FIG. 9.

Alternatively, in the case where the M line 13e is pre-marked in the B-mode image 11, the user may select the M line 13e using the input unit 160, and the display controller 180 may then move the B-mode image 11 to the left to fully display the selected M line 13e on the screen.

As another alternative exemplary embodiment, the user may personally designate the position of the M line 13e using the input unit 160. When the designated position is not fully shown on the screen, the display controller 180 may move the B-mode image 11 to fully display the M line 13e on the screen.

On the other hand, when the M line 13e is additionally set, the M-mode image 14 displayed on the screen is updated to be added with an M-mode image corresponding to the additionally set M line 13e.

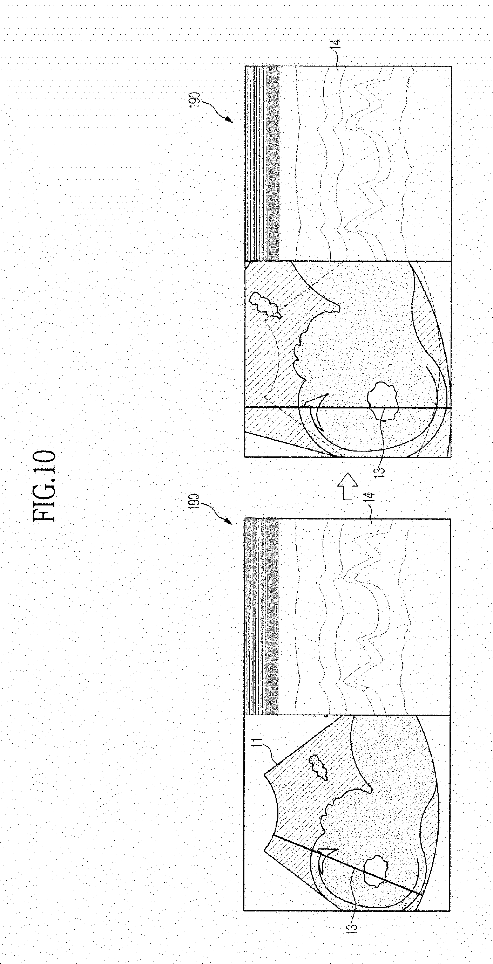

FIG. 10 illustrates images of a sixth example displayed on the display unit 190 of the ultrasonic diagnostic apparatus 100 according to the exemplary embodiment of the present invention.

In the case where a set M line 13 and a corresponding M-mode image 14 are displayed as shown at the left side of FIG. 10, that is, a vertical axis of the M-mode image 14 and the M line 13 are not parallel with each other, the display controller 180 may rotate a B-mode image 11 such that the M line 13 and the vertical axis are parallel with each other. At this time, the rotation angle of the B-mode image 11 will be an angle defined as extending between a vertical scan line and the M line 13.

Also, the display controller 180 may scale the B-mode image 11 such that the depth ratio of the M-mode image 14 to the B-mode image 11 is 1:1.

In the case that the display controller 180 rotates and scales the B-mode image 11 such that the M line 13 and the vertical axis of the M-mode image 14 are parallel with each other and the depth ratio of the two images is 1:1, as shown at the right side of FIG. 10, the user may be able to readily and accurately compare/analyze the B-mode image 11 and the M-mode image 14.

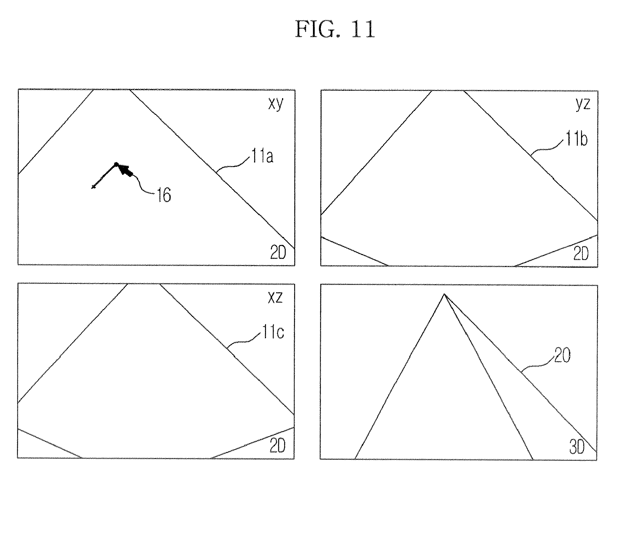

FIG. 11 illustrates examples of images 11a-11c displayed on a display unit 190 of an ultrasonic diagnostic apparatus 100 according to an alternative exemplary embodiment of the present invention.

The ultrasonic diagnostic apparatus 100 according to the alternative exemplary embodiment of the present invention may generate and display a two-dimensional ultrasonic image and a three-dimensional ultrasonic image. In this case, the first mode image generator 150 in the block diagram of FIG. 1 may be a two-dimensional ultrasonic image generator and the second mode image generator 170 therein may be a three-dimensional ultrasonic image generator. Other components are substantially the same as those in FIG. 1.

Referring to FIG. 11, a two-dimensional (2D) ultrasonic image (B-mode image) of each section of a three-dimensional (3D) ultrasonic image 20 may be displayed on the display unit 190 together with the 3D ultrasonic image 20. The 2D ultrasonic image of each section may include a cross-sectional image 11a in an xy plane, a cross-sectional image 11b in a yz plane, and a cross-sectional image 11c in an xz plane, as well as the 3D ultrasonic image 20 shown in a perspective view 30.

Here, when the user manipulates the input unit 160 to input a command for image movement, the display controller 180 moves an image in response to the input command.

As an example, when the user manipulates the input unit 160 to move the cursor 16 on the cross-sectional image 11a in the xy plane, the display controller 180 may change, translate, rotate, and/or move the cross-sectional image 11a according to a moving amount and moving direction of the cursor 16 and also move the 3D ultrasonic image 20 and the other two cross-sectional images 11b and 11c corresponding to the movement of the cross-sectional image 11a, thereby allowing the user to readily compare/analyze data of the respective images.

On the other hand, when the user manipulates the input unit 160 to move the 3D ultrasonic image 20, the display controller 180 may move the 2D ultrasonic images 11a, 11b and 11c corresponding to the movement of the 3D ultrasonic image 20.

Alternatively, in the case where the input unit 160 is implemented with a trackball or mouse, the user may click the trackball or mouse on a desired point of an image to designate the desired point, and then manipulate the trackball or mouse to move the cursor 16 to a next point so as to move the image.

Hereinafter, exemplary embodiments of a method of controlling the ultrasonic diagnostic apparatus 100 according to the exemplary embodiment of the present invention will be described.

The method of controlling the ultrasonic diagnostic apparatus according to the exemplary embodiment of the present invention includes generating an image of a first mode and an image of a second mode using an ultrasonic signal reflected and returned after being transmitted to an object, displaying the generated first mode image and second mode image on the display unit 190, receiving a command for image movement input by the user, and moving one of the first mode image and second mode image on the screen in response to the input command to display a portion initially omitted on the screen. The exemplary embodiments of the ultrasonic diagnostic apparatus control method will hereinafter be described in detail with reference to the annexed drawings. In the following description, in an illustrative example, the first mode is the B-mode and the second mode is the M-mode.

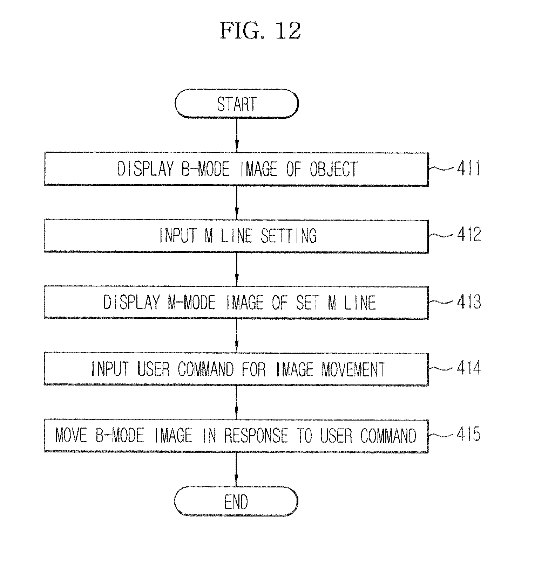

FIG. 12 is a flowchart illustrating a method of controlling the ultrasonic diagnostic apparatus 100 according to the exemplary embodiment of the present invention, which describes, for example, the control of the images shown in FIGS. 6A-6B.

Referring to FIG. 12, first, a B-mode image of an object is generated and displayed on the display unit 190 in step 411. Generating the B-mode image is performed in the same manner as that previously stated in association with the ultrasonic diagnostic apparatus 100, and a description thereof will thus be omitted.

Then, a setting of an M line input by the user is received in step 412. Alternatively, the setting of the M line may be made directly by the ultrasonic diagnostic apparatus 100, and not by the user input. If the M line is set, the M line is marked in the B-mode image.

When the M line is set, an M-mode image of the set M line is generated and displayed on the display unit 190 in step 413. Generating the M-mode image is also performed in the same manner as that previously stated in association with the ultrasonic diagnostic apparatus 100, and a description thereof will thus be omitted.

The B-mode image and the M-mode image are displayed on one screen, as shown in, for example, FIG. 6A. When a part of the M line marked in the B-mode image is omitted so as not to be initially shown on the screen, a user command for image movement is input using the input unit 160 in step 414. At this time, the user command input is performed by manipulation of the input unit 160 by the user.

Then, the B-mode image is moved in response to the user command in step 415 such that the partially omitted M line is fully shown on the screen, and the method of FIG. 12 ends. In particular, in the case where the input unit 160 is a trackball or mouse, the user may move the cursor 16 displayed on the B-mode image toward an omitted portion of the M line by rolling the trackball or moving the mouse. The B-mode image may be moved with the movement of the cursor 16 such that the omitted portion of the M line is shown on the screen. In the case where the input unit 160 is a keyboard, the user may move the cursor 16 displayed on the B-mode image using direction keys. In the case where the input unit 160 is a touch pad or touch screen, the user may move the cursor 16 by personally touching the touch pad or touch screen.

The moving direction of the B-mode image may be as follows. When the partially omitted M line is located at the left-hand side of a vertical scan line, the B-mode image may be moved to the right. Otherwise, when the partially omitted M line is located at the right-hand side of the vertical scan line, the B-mode image may be moved to the left. When upper and lower portions of the B-mode image are also initially omitted, the B-mode image may also be moved up and down.

The moving amount of the B-mode image may be as follows. The B-mode image may be moved until the partially omitted M line is fully or completely displayed on the screen. Alternatively, the B-mode image may be moved according to a moving amount input using the input unit 160.

The B-mode image may be moved in an M line setting process, which will hereinafter be described with reference to an example in which the user personally draws the M line. First, the B-mode image of the object is generated and displayed on the display unit 190. The user designates a point on the B-mode image to be set as a start point of the M line and then draws the M line toward an end point of the M line. At this time, an area of the B-mode image in which the M line is to be drawn may be partially displayed on the screen. In this case, the B-mode image may be moved at the same time that the M line is drawn toward a portion of the area, which is not initially displayed, such that the area portion is displayed on the screen.

As another example of moving the B-mode image in the M line setting process, the B-mode image of the object is generated and displayed on the display unit 190, and at least one M line is pre-marked in the B-mode image. The user may perform the M line setting by selecting the pre-marked M line. When the M line selected by the user is partially shown on the screen, the B-mode image may be moved such that the selected M line is fully shown on the screen.

As another example of moving the B-mode image in the M line setting process, the B-mode image of the object is generated and displayed on the display unit 190, and the user sets the M line by designating the position of the M line in the B-mode image. The set M line is displayed at the designated position of the B-mode image. At this time, in the example in which a part of the designated position is not initially displayed on the screen, the B-mode image may be moved such that the set M line is fully shown on the screen.

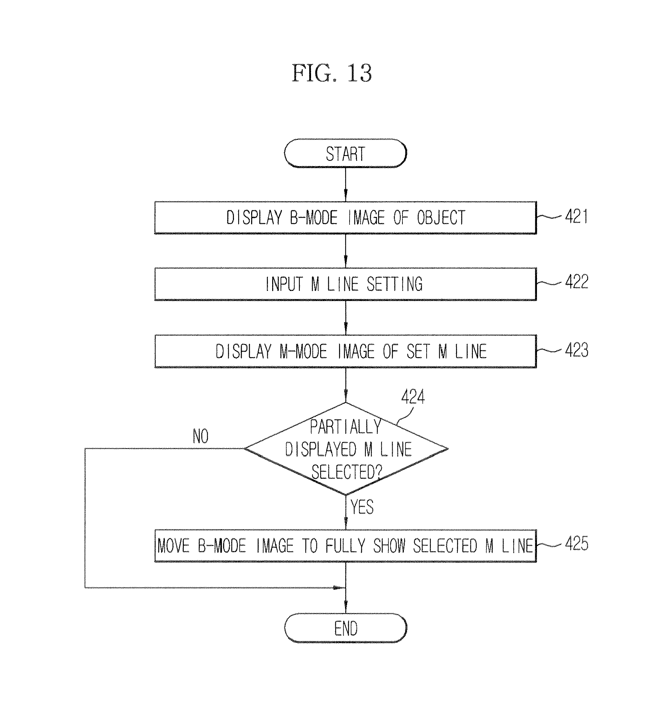

FIG. 13 is a flowchart illustrating an example of automatic image movement, which describes, for example, the control of the images shown in FIGS. 7A-7B.

Referring to FIG. 13, first, a B-mode image of an object is displayed on the display unit 190 in step 421 and a setting of an M line input by the user is received in step 422. Then, an M-mode image corresponding to the set M line is generated and displayed on the display unit 190 in step 423. Here, the B-mode image and the M-mode image are displayed on one screen, and the set M line may include at least one M line, such as a plurality of M lines.

At this time, the set M line may be partially displayed on the screen. When a selection for the partially displayed M line is determined to have been input by the user in step 424, the B-mode image is moved such that the selected M line is fully displayed on the screen in step 425, and the method in FIG. 13 ends. At this time, the moving amount and moving direction of the B-mode image are determined depending on a hidden position and hidden degree of the M line. However, if no selection for the partially displayed M line has been input, as determined in step 424, the method skips step 425 and the method in FIG. 13 ends.

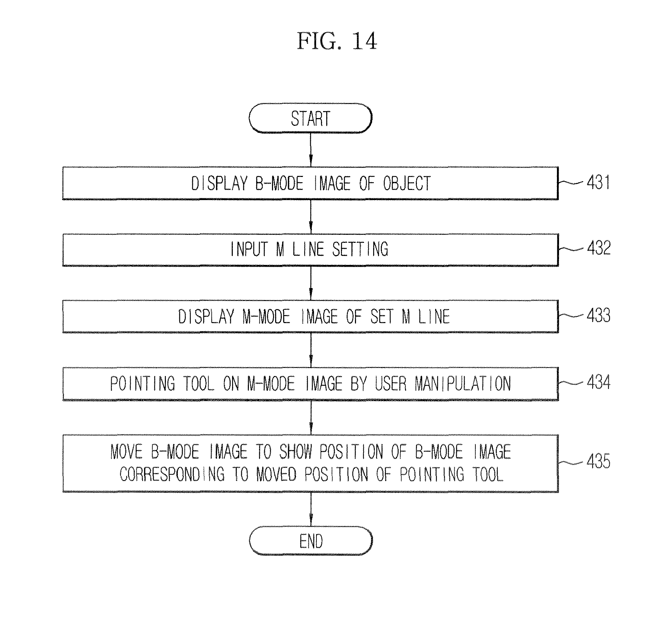

FIG. 14 is a flowchart illustrating an example of movement of a B-mode image based on the position of a pointing tool, which describes, for example, the control of the images shown in FIG. 8.

Referring to FIG. 14, first, a B-mode image of an object is generated and displayed on the display unit 190 in step 431 and a setting of an M line input by the user is received in step 432. The set M line is marked in the B-mode image.

An M-mode image of the set M line is generated and displayed on the display unit 190 in step 433, and the user moves the pointing tool, for example, the cursor 16 on the M-mode image, as shown in FIG. 6A, by manipulating the input unit 160 in step 434. The movement of the pointing tool based on the user manipulation is performed through manipulation of the input unit 160 by the user. For example, in the case where the input unit 160 is a trackball or mouse, the moving direction and moving amount of the pointing tool are input by the user rolling the trackball or moving the mouse. In the case where the input unit 160 is a keyboard, the moving direction and moving amount of the pointing tool are input by the user manipulating buttons of the keyboard. In the case where the input unit 160 is a touch panel or touch screen, the moving direction and moving amount of the pointing tool are input by the user touching the touch panel or touch screen.

When a position of the B-mode image corresponding to the moved position of the pointing tool is not initially shown on the screen, the B-mode image is moved such that the corresponding position is shown on the screen in step 435, and the method of FIG. 14 ends. The moving direction and moving amount of the B-mode image are determined depending on the moved position of the pointing tool.

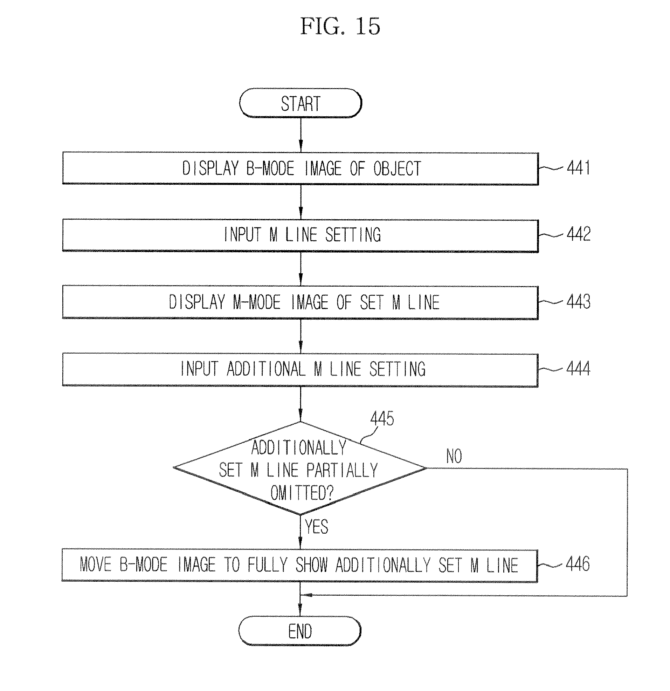

FIG. 15 is a flowchart illustrating an example of movement of a B-mode image based on an additional setting of an M line, which describes, for example, the control of the images shown in FIG. 9.

Referring to FIG. 15, first, a B-mode image of an object is generated and displayed on the display unit 190 in step 441 and a setting of an M line input by the user is received in step 442. If the M line setting is input, an M-mode image of the set M line is generated and displayed on the display unit 190 in step 443. The B-mode image and the M-mode image are displayed on one screen of the display unit 190, as shown, for example, in FIG. 6A.

Then, an additional setting of an M line input by the user is received in step 444. When the additionally set M line is partially omitted on the screen as determined in step 445, the B-mode image is moved such that the additionally set M line is fully displayed on the screen in step 446, and the method of FIG. 15 ends. Otherwise if it is determined in step 445 that no additionally set M line is partially omitted on the screen, the method skips step 446 and then the method of FIG. 15 ends. The additional M line setting may be made by the user personally drawing the M line on the B-mode image or designating the position of the M line. The moving direction and moving amount of the B-mode image are determined depending on the omitted degree and position of the additionally set M line.

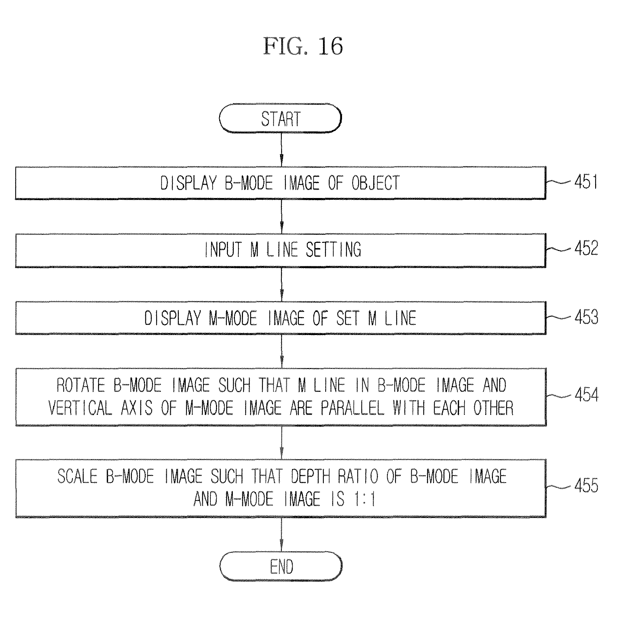

FIG. 16 is a flowchart illustrating an example of rotation and scaling of a B-mode image, which describes, for example, the control of the images shown in FIG. 10.

Referring to FIG. 16, first, a B-mode image of an object is generated and displayed on the display unit 190 in step 451 and a setting of an M line input by the user is received in step 452. The setting of the M line may be made directly by the ultrasonic diagnostic apparatus 100, not by the user input.

If the M line is set, an M-mode image of the set M line is generated and displayed on the display unit 190 in step 453.

In the case where the set M line is not a vertical scan line of the B-mode image, it may not be easy for the user to compare/analyze the B-mode image and the M-mode image. For this reason, the B-mode image is rotated such that the M line marked in the B-mode image and a vertical axis of the M-mode image are parallel with each other in step 454.

Then, the B-mode image is scaled such that the depth ratio of the two images is 1:1 in step 455, and the method of FIC. 16 then ends. Therefore, the user may readily compare/analyze the B-mode image and the M-mode image.

A method of controlling the ultrasonic diagnostic apparatus 100 according to an alternative exemplary embodiment of the present invention includes generating a two-dimensional ultrasonic image and a three-dimensional ultrasonic image with respect to an object, as described herein with regard to FIG. 11, displaying the generated images on one screen, moving the two-dimensional ultrasonic image or three-dimensional ultrasonic image in response to a user input, and moving the other image together corresponding to the image movement. For example, an xy cross-sectional image 11a, a yz cross-sectional image 11b, and an xz cross-sectional image 11c of a three-dimensional ultrasonic image may be displayed on one screen together with the three-dimensional ultrasonic image 30. If the user moves one of the above images by moving the position of the cursor 16 using the input unit 160, the other images may also be moved corresponding to the image movement.

As described above, when images corresponding to two types of diagnostic modes are simultaneously displayed on the display unit of the ultrasonic diagnostic apparatus 100, a portion of the displayed images may be initially omitted or hidden. In this case, the omitted or hidden portion may be displayed on the screen by a simple operation of the user. Therefore, the user may compare/analyze two types of data corresponding to each other on one screen, so as to more efficiently and accurately perform an ultrasonic diagnosis.

Alternatively, the ultrasonic diagnostic apparatus 100 may automatically display the omitted or hidden portion on the screen without a user operation. Therefore, the two types of data may be conveniently compared/analyzed without an increase in the number of operation steps of the user.

As is apparent from the above description, in an ultrasonic diagnostic apparatus 100 and a control method thereof according to the exemplary embodiment of the present invention, when two or more types of ultrasonic data are displayed on one screen, it may be possible to prevent the data from being reduced in size or being completely omitted on the screen, thereby performing an accurate diagnosis through comparison of the data.

Also, data omitted on the screen may be displayed through a simple operation of the user, thereby reducing the number of operating steps of the user. Further, the user may perform data comparison/analysis by intuition.

The above-described apparatus and methods according to the present invention can be implemented in hardware or firmware, or as software or computer code, or combinations thereof. In addition, the software or computer code can also be stored in a non-transitory recording medium such as a CD ROM, a RAM, a ROM whether erasable or rewritable or not, a floppy disk, CDs, DVDs, memory chips, a hard disk, a magnetic storage media, an optical recording media, or a magnetooptical disk or computer code downloaded over a network originally stored on a remote recording medium, a computer readable recording medium, or a non-transitory machine readable medium and to be stored on a local recording medium, so that the methods described herein can be rendered in such software, computer code, software modules, software objects, instructions, applications, applets, apps, etc. that is stored on the recording medium using a general purpose computer, a digital computer, or a special processor or in programmable or dedicated hardware, such as an ASIC or FPGA. As would be understood in the art, the computer, the processor, microprocessor controller or the programmable hardware include volatile and/or non-volatile storage and memory components, e.g., RAM, ROM, Flash, etc. that may store or receive software or computer code that when accessed and executed by the computer, processor or hardware implement the processing methods described herein. In addition, it would be recognized that when a general purpose computer accesses code for implementing the processing shown herein, the execution of the code transforms the general purpose computer into a special purpose computer for executing the processing shown herein. In addition, the program may be electronically transferred through any medium such as communication signals transmitted by wire/wireless connections, and their equivalents. The programs and computer readable recording medium can also be distributed in network-coupled computer systems so that the computer readable code is stored and executed in a distributed fashion.

Although a few exemplary embodiments of the present invention have been shown and described, it would be appreciated by those skilled in the art that changes may be made in these exemplary embodiments without departing from the principles of the invention, the scope of which is defined in the claims and their equivalents.

## Claims

1. An ultrasonic diagnostic apparatus (100) including a probe (110) configured to emit an ultrasonic signal to an object and receive the ultrasonic signal reflected from the object and a first mode image generator (150) configured to generate an image (11) of a first mode based on the reflected ultrasonic signal received by the probe (110), the ultrasonic diagnostic apparatus **characterized by**:
a second mode image generator (170) configured to generate an image (14) of a second mode associated with the first mode image;
a display unit (190) configured to display the first mode image and the second mode image on one screen thereof; and
a display controller (180) configured to move, when a part of a portion of the first mode image (11) associated with the second mode image (14) is not displayed on the display unit, the first mode image (11) displayed on the display unit (190) to fully display the portion associated with the second mode image (14) on the display unit (190).

2. The ultrasonic diagnostic apparatus (100) according to claim 1, **characterized in that** the first mode image (11) is a B-mode image having at least one M line (13) marked therein; and
the second mode image (14) is an M-mode image corresponding to the at least one M line (13) marked in the B-mode image (11).

3. The ultrasonic diagnostic apparatus (100) according to claim 2, **characterized in that** the display controller (180) is configured to move, when a portion of the M line (13) is not displayed on the display unit (190), the B-mode image (11) to display the M line portion (13) on the display unit (190).

4. The ultrasonic diagnostic apparatus (100) according to claims 2 or 3, **characterized by** an input unit (160) configured to receive a command for movement of the B-mode image (11) displayed on the display unit (190); and
the display controller (180) being configured to move the B-mode image (11) displayed on the display unit (190) based on a moving direction and a moving amount corresponding to the command received from the input unit (160).

5. The ultrasonic diagnostic apparatus (100) according to claim 4, **characterized in that** the display unit (190) is configured to display a pointing tool (16), with the pointing tool (16) being moved in response to the command received from the input unit (160); and
the display controller (180) being configured to move, when a portion of the M line (13) located in a moving direction of the pointing tool (16) is not displayed on the display unit (190), the B-mode image (11) displayed on the display unit (190) to display the M line portion (13) on the display unit (190).

6. The ultrasonic diagnostic apparatus (100) according to claim 4, **characterized in that** the display unit (190) is configured to display a pointing tool (16), with the pointing tool (16) being moved in response to the command received from the input unit (160); and
the display controller (180) being configured to move, when a portion of the M line (13) corresponding to a portion of the M-mode image (14) in which the pointing tool (16) moved in response to the command is located is not displayed on the display unit (190), the B-mode image (11) to display the M line portion (13) on the display unit (190).

7. The ultrasonic diagnostic apparatus (100) according to any of the claims 2 - 6, **characterized by** an input unit (160) configured to receive an inputted setting of the M line (13b); and
the display controller (180) being configured to move, when an additional setting of an M line (13a) is received from the input unit (160) and a portion of the additionally set M line (13a) is not displayed on the display unit (190), the B-mode image (11) displayed on the display unit (190) to fully display the additionally set M line (13a).

8. The ultrasonic diagnostic apparatus (100) according to claim 7, **characterized in that** the display controller (190) is configured to additionally display an M-mode image (14) corresponding to the additionally set M line (13a) on the display unit (190) when the additional M line (13a) setting is completed.

9. The ultrasonic diagnostic apparatus (100) according to any of the claims 2 - 8, **characterized in that** the display controller (180) is configured to rotate the B-mode image (11) such that the M line (13) marked in the B-mode image (11) is parallel with a vertical axis of the M-mode image (14).

10. The ultrasonic diagnostic apparatus (100) according to claim 9, **characterized in that** the display controller (180) is configured to scale the rotated B-mode image (11) displayed on the display unit (190) such that a depth ratio of the B-mode image (11) to the M-mode image (14) is 1:1.

11. The ultrasonic diagnostic apparatus (100) according to claim 7 or 8, **characterized in that** the input unit (160) includes at least one of a mouse, a trackball, a keyboard, a touch panel and a touch screen.

12. A method of controlling an ultrasonic diagnostic apparatus (100) including emitting an ultrasonic signal to an object and receiving the ultrasonic signal reflected from the object and generating an image (11) of a first mode based on the received reflected ultrasonic signal , the method **characterized by**:
generating an image (14) of a second mode associated with the first mode image (11);
displaying (413) the generated second mode image together with the first mode image (11);
moving (415) the displayed first mode image (11) when a part of a portion of the first mode image (11) associated with the second mode image (14) is not displayed, to fully display the portion associated with the second mode image (14).

13. The method according to claim 11, **characterized in that** the first mode image (11) is a B-mode image having at least one M line (13) marked therein; and
the second mode image (14) is an M-mode image corresponding to the at least one M line (13).

14. The method according to claim 12, **characterized in that** the moving (415) of the displayed first mode image (11) comprises moving (425) the B-mode image (11) when a portion of the M line (13) is not displayed, to display the M line portion (13).

15. The method according to any of the claims 12 - 14, **characterized by** receiving (414) a command for image movement; and
the moving (415) of the displayed first mode image (11) comprises moving (415) the displayed B-mode image (11) based on a moving amount and a moving direction corresponding to the received command.
